# EUROPEAN PATENT APPLICATION

(11) **EP 3 766 985 A1**
(43) Date of publication of application: **20.01.2021**
(21) Application number: 19767182.9
(22) Date of filing: 11.03.2019
(51) Int. Cl.: C12Q 1/04, C12N 5/071, C12N 15/09, C12Q 1/6841, C12Q 1/6851

(54) **IDENTIFICATION METHOD FOR DERMAL SHEATH CUP CELLS (DSCC), AND METHOD FOR EVALUATING COMPOSITION FOR HAIR FOLLICLE REGENERATION**

(30) Priority: 12.03.2018 JP 2018044262
(71) Applicant: Shiseido Company, Ltd., Chuo-ku Tokyo 104-0061 (JP)
(72) Inventor: ISHIMATSU, Yumiko, Yokohama-shi, Kanagawa 224-8558 (JP); SOMA, Tsutomu, Yokohama-shi, Kanagawa 224-8558 (JP); KISHIMOTO, Jiro, Yokohama-shi, Kanagawa 224-8558 (JP)
(74) Representative: Santarelli
(86) International application number: PCT/JP2019/009798
(87) International publication number: WO 2019/176881

(57) **Abstract**

Provided is an identification method for dermal sheath cup cells (DSCC) in hair follicle-derived cells, said method being characterized by comprising using the expression level of GREM2 gene as an index. Also provided is a method for evaluating a composition for hair follicle regeneration which contains hair follicle-derived cells, said method being characterized by comprising determining the ratio and/or activity of dermal sheath cup cells (DSCC) in the hair follicle-derived cells with the use of the expression level of GREM2 gene as an index.

## Description

### FIELD

The present invention relates to a method for identifying dermal sheath cup (DSC) cells in a hair follicle-derived cell group. The present invention further relates to a method for evaluating compositions for regenerating hair follicles containing the hair follicle-derived cell group.

### BACKGROUND

Hair is produced by hair follicles present in the skin. A hair follicle is a tissue layer that surrounds hair and is composed of ectoderm-derived hair matrix (hair matrix cells), inner root sheath, outer root sheath, mesoderm-derived dermal sheath, and dermal papilla, etc. Hair matrix cells that surround dermal papilla are induced to divide repeatedly by being supplied with nutrients and proteins from the dermal papilla and form hair through the keratinization thereof.

The occurrence of problems with hair growth causes hair loss and alopecia. Hair loss and alopecia include androgenetic alopecia, alopecia areata, telogen effluvium, etc., with the most common being androgenetic alopecia. Androgenetic alopecia arises mainly from the effects of male hormones in men and is also called male pattern baldness. Hair regrowth occurs through the repetition of the hair cycle which consists of anagen, catagen and telogen stages. The symptoms of male pattern baldness occur due to the anagen stage becoming shorter within the hair cycle and due to an increase in the proportion of thin short hair.

Presently, the topical agent minoxidil and the orally administered finasteride are mainly used for the treatment for androgenetic alopecia. These treatment methods have both been confirmed to be effective and safe but are not always effective against all cases of androgenetic alopecia.

Other treatments for androgenetic alopecia include autologous hair transplantation. Autologous hair transplantation is a technique whereby hair including the hair root is taken from the temporal or occipital region of the scalp then transplanted into a bald region of the same individual. However, since this technique transplants hair onto a different place of the same individual it does not increase the total amount of hair.

Recently, regenerative medical techniques are being developed for various ailments and this is also the case in the field of hair regeneration where research and development of new techniques are being carried out. For example, it has been suggested that within the hair follicle, the dermal sheath located in the outermost layer of the hair follicle, in particular, dermal sheath cup (DSC) cells located at the bottom part of the hair bulb have a high hair follicle-inductive capacity, and it has been reported that in experiments in which DSC cells were transplanted, hair growth was confirmed (NPL 1 and 2). It has also been shown that DSC cells are progenitor cells of dermal papilla (DP) cells which play an important role in hair growth and DSC cells have been receiving attention (NPL 3).

### [CITATION LIST]

### [NON-PATENT LITERATURE]

[NPL 1] Jahoda CA et al., Induction of hair growth by implantation of cultured dermal papilla cells. Nature 1984; 311:560-562
[NPL 2] McElwee KJ et al., Culture peribulbar dermal sheath cells can induce hair follicle development and contribute to the dermal sheath and dermal papilla. J. Invest. Dermatol. 2003; 121: 1267-1275
[NPL 3] Rahmani W. et al., Hair follicle dermal stem cells regenerate the dermal sheath, repopulate the dermal papilla, and modulate hair type. Dev. Cell. 2014 Dec. 8; 31 (5): 543-58.

### SUMMARY

### [TECHNICAL PROBLEM]

In the field of hair regeneration, treatment methods using hair follicle-derived cell groups are being developed but presently, the only method for obtaining dermal sheath cup cells, which have the capacity to induce hair follicles, is by visually collecting cells from around the bottom of the hair bulb and no specific markers are known. Further, it was not possible to evaluate the number of non-dermal sheath cup cells mixed in the collected cells. Furthermore, after *in vitro* culturing of the collected dermal sheath cup cells, it was not possible to evaluate the number of surviving cells that had retained this capacity.

Thus, the object of the present invention is to elucidate a marker gene that is specifically expressed in a hair follicle-derived cell group, in particular, in dermal sheath cup cells, and provide a method for identifying dermal sheath cup cells using the expression level of the gene as an indicator. The object is also to provide a method for evaluating compositions comprising a hair follicle-derived cell group for regenerating hair follicles.

### [SOLUTION TO PROBLEM]

As a result of extensive research carried out by the present inventors, genes expressed in dermal sheath cup cells were discovered and thereby the present invention was completed. Specifically, the present invention encompasses the following.

[1] A method for identifying dermal sheath cup (DSC) cells in a hair follicle-derived cell group, characterized by using the expression level of the *GREM2* gene as an indicator.
[2] The method according to [1], characterized by further using the expression level of one or more genes selected from the group consisting of the *ASPN, MMP11, PTGER3, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[3] The method according to [1] or [2] wherein the hair follicle-derived cell group has been cultured *in vitro.*
[4] The method according to [3] wherein the hair follicle-derived cell group has been subcultured at least once.
[5] A method for evaluating a composition for regenerating hair follicles containing a hair follicle-derived cell group, characterized by determining the ratio and/or activity of dermal sheath cup (DSC) cells in the hair follicle-derived cell group using the expression level of the *GREM2* gene as an indicator.
[6] The method according to [5], characterized by further using the expression level of one or more genes selected from the group consisting of the *ASPN, MMP11, PTGER3, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[7] The method according to [5] or [6] wherein the hair follicle-derived cell group has been cultured *in vitro.*
[8] The method according to [7] wherein the hair follicle-derived cell group has been subcultured at least once.
[9] A method for identifying dermal sheath cup (DSC) cells in a hair follicle-derived cell group characterized by using the expression level of the *ASPN* gene as an indicator.
[10] The method according to [9] characterized by further using the expression level of one or more genes selected from the group consisting of the *GREM2, MMP11, PTGER3, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[11] The method according to [9] or [10] wherein the hair follicle-derived cell group has been cultured *in vitro.*
[12] The method according to [11] wherein the hair follicle-derived cell group has been subcultured at least once.
[13] A method for evaluating a composition for regenerating hair follicles containing a hair follicle-derived cell group, characterized by determining the ratio and/or activity of dermal sheath cup (DSC) cells in the hair follicle-derived cell group using the expression level of the *ASPN* gene is used as an indicator.
[14] The method according to [13] characterized by further using the expression level of one or more genes selected from the group consisting of the *GREM2, MMP11, PTGER3, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[15] The method according to [13] or [14] wherein the hair follicle-derived cell group has been cultured *in vitro.*
[16] The method according to [15] wherein the hair follicle-derived cell group has been subcultured at least once.
[17] A method for identifying dermal sheath cup (DSC) cells in a hair follicle-derived cell group characterized by using the expression level of the *MMP11* gene as an indicator.
[18] The method according to [17], characterized by further using the expression level of one or more genes selected from the group consisting of the *GREM2, ASPN, PTGER3, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L and PCSK7* as the indicator.
[19] The method according to [17] or [18] wherein the hair follicle-derived cell group has been cultured *in vitro.*
[20] The method according to [19] wherein the hair follicle-derived cell group has been subcultured at least once.
[21] A method for evaluating a composition containing a hair follicle-derived cell group for regenerating hair follicles, characterized by determining the ratio and/or activity of dermal sheath cup (DSC) cells in the hair follicle-derived cell group using the expression level of the *MMP11* gene as an indicator.
[22] The method according to [21], characterized by further using the expression level of one or more genes selected from the group consisting of the *GREM2, ASPN, PTGER3, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[23] The method according to [21] or [22] wherein the hair follicle-derived cell group has been cultured *in vitro.*
[24] The method according to [23] wherein the hair follicle-derived cell group has been subcultured at least once.
[25] A method for identifying dermal sheath cup (DSC) cells in a hair follicle-derived cell group, characterized by using the expression level of the *PTGER3* gene as an indicator.
[26] The method according to [25], characterized by further using the expression level of one or more genes selected from the group consisting of the *GREM2, ASPN, MMP11, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[27] The method according to [25] and [26] wherein the hair follicle-derived cell group has been cultured *in vitro.*
[28] The method according to [27] wherein the hair follicle-derived cell group has been subcultured at least once.
[29] A method for evaluating a composition for regenerating hair follicles containing a hair follicle-derived cell group, characterized by determining the ratio and/or activity of dermal sheath cup (DSC) cells in the hair follicle-derived cell group using the expression level of *PTGER3* gene as an indicator.
[30] The method according to [29], characterized by further using the expression level of one or more genes selected from the group consisting of the *GREM2, ASPN, MMP11, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[31] The method according to [29] or [30] wherein the hair follicle-derived cell group has been cultured *in vitro.*
[32] The method according to [31] wherein the hair follicle-derived cell group has been subcultured at least once.
[33] A method for identifying dermal sheath cup (DSC) cells in a hair follicle-derived cell group, characterized by using the expression level of the *RBP4* gene as an indicator.
[34] The method according to [33], characterized by further using the expression level of one or more genes selected from the group consisting of the *GREM2, ASPN, MMP11, PTGER3, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[35] The method according to [33] and [34] wherein the hair follicle-derived cell group has been cultured *in vitro.*
[36] The method according to [35] wherein the hair follicle-derived cell group has been subcultured at least once.
[37] A method for evaluating a composition for regenerating hair follicles containing a hair follicle-derived cell group, characterized by determining the ratio and/or activity of dermal sheath cup (DSC) cells in the hair follicle-derived cell group using the expression level of the *RBP4* gene as an indicator.
[38] The method according to [37], characterized by further using the expression level of one or more genes selected from the group consisting of the *GREM2, ASPN, MMP11, PTGER3, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[39] The method according to [37] or [38] wherein the hair follicle-derived cell group has been cultured *in vitro.*
[40] The method according to [39] wherein the hair follicle-derived cell group has been subcultured at least once.
[41] A method for identifying dermal sheath cup (DSC) cells in a hair follicle-derived cell group, characterized by using the expression level of the *TLE4* gene as an indicator.
[42] The method according to [41], characterized by further using the expression level of one or more genes selected from the group consisting of the *GREM2, ASPN, MMP11, PTGER3, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[43] The method according to [41] or [42] wherein the hair follicle-derived cell group has been cultured *in vitro.*
[44] The method according to [43] wherein the hair follicle-derived cell group has been subcultured at least once.
[45] A method for evaluating a composition containing a hair follicle-derived cell group for regenerating hair follicles, characterized by determining the ratio and/or activity of dermal sheath cup (DSC) cells in the hair follicle-derived cell group using the expression level of the *TLE4* gene an indicator.
[46] The method according to [45], characterized by further using the expression level of one or more genes selected from the group consisting of the *GREM2, ASPN, MMP11, PTGER3, RBP4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.
[47] The method according to [45] or [46] wherein the hair follicle-derived cell group has been cultured *in vitro.*
[48] The method according to [47] wherein the hair follicle-derived cell group has been subcultured at least once.

### [ADVANTAGEOUS EFFECTS OF INVENTION]

The present invention allows dermal sheath cup cells contained in a hair follicle-derived cell group to be identified and allows the quality of a composition for regenerating hair follicles containing a hair follicle-derived cell group to be evaluated. Furthermore, the invention can evaluate whether dermal sheath cup cells have remained after isolated dermal sheath cup cells are cultured and proliferated *in vitro,* and allows the selection of a composition with a high hair follicle-inductive capacity for regenerating hair follicles.

### BRIEF DESCRIPTION OF DRAWINGS

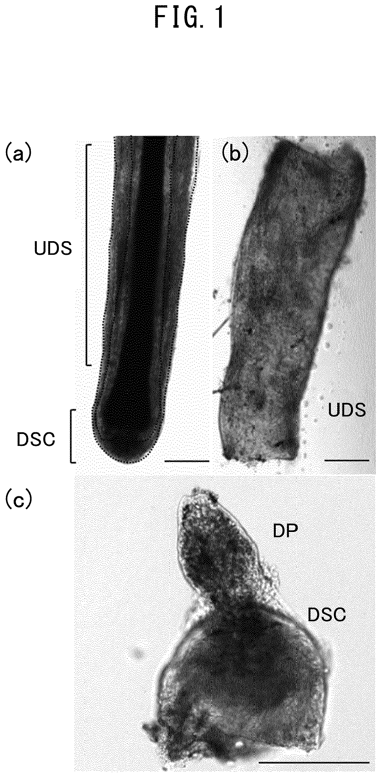

[FIG. 1] is a picture showing hair follicle (HF) subunits. (a) A whole HF. (b) Dissected upper dermal sheath (UDS). (c) Dissected and inverted dermal sheath cup (DSC) and dermal papilla (DP) before separation. Scale bar = 200 µm.

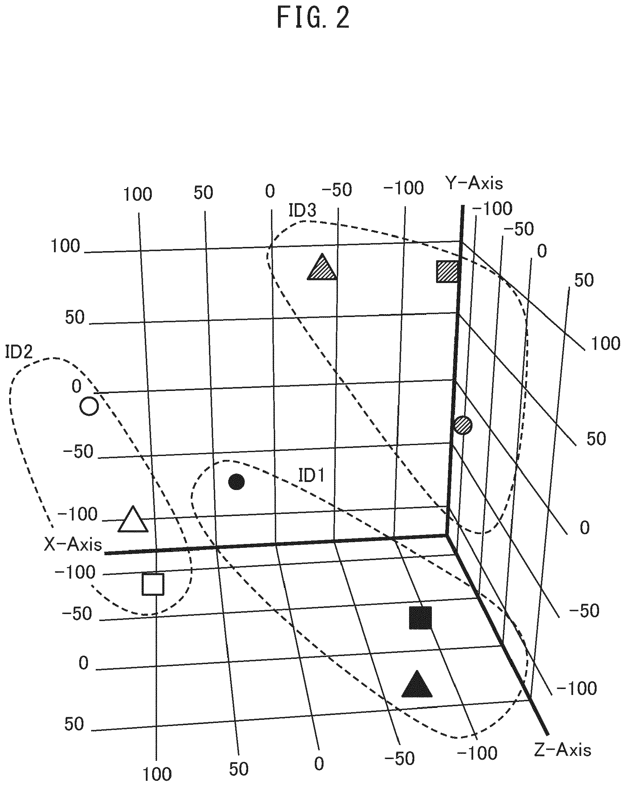

[FIG. 2] is a graph illustrating the similarity of the microarray results after principle component analysis and is a three-dimensional view from different viewpoints. The X-axis represents the principal component (PC) 1 (32.34% variance captured); the Y-axis represent PC2 (22.83% variance captured); the Z-axis represents PC3 (20.43% variance captured). The black symbols represent individual (ID) 1; the white symbols represent ID2; and the striped symbols represent ID3. The triangles represent DSC, the squares represent DP, and the circles represent UDS.

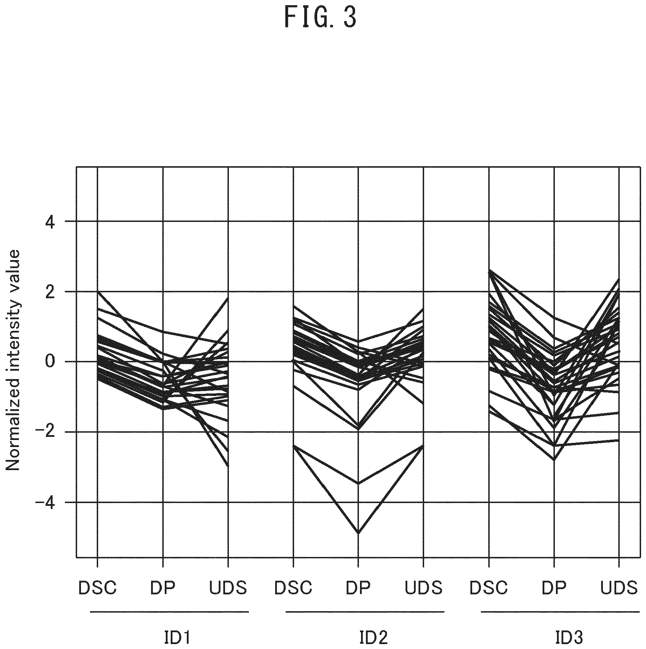

[FIG. 3] illustrates genes that are expressed differently in the dermal sheath cup (DSC). Normalized expression levels of DSC marker genes were compared with dermal papilla (DP) and upper dermal sheath (UDS) in each of three individuals.

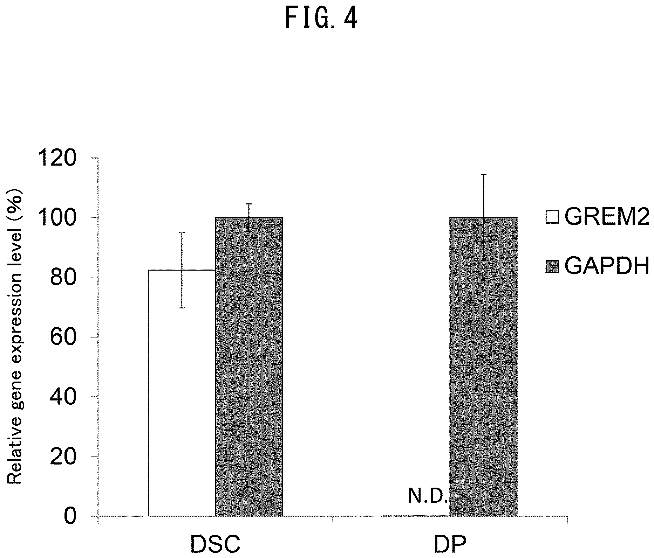

[FIG. 4] is a graph illustrating the expression level of *GREM2* in the dermal sheath cup (DSC) and dermal papilla (DP) as analyzed by quantitative PCR. The value relative to when *GAPDH* expression is 100% is shown.

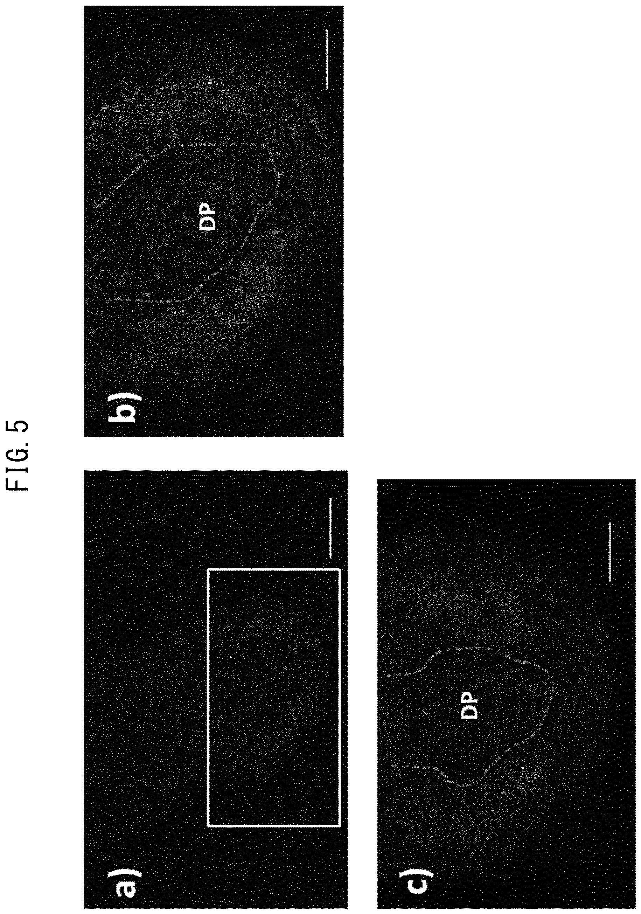

[FIG. 5] shows pictures illustrating the expression of *GREM2* in human hair follicles and shows the results of *in situ* hybridization. (a) *GREM2* probe, (b) an enlarged view of (a), (c) no probe. Red: *in situ* signal; Blue: nuclear staining with Hoechst 33342. Scale bar (a): 100 µm, (b) and (c): 50 µm.

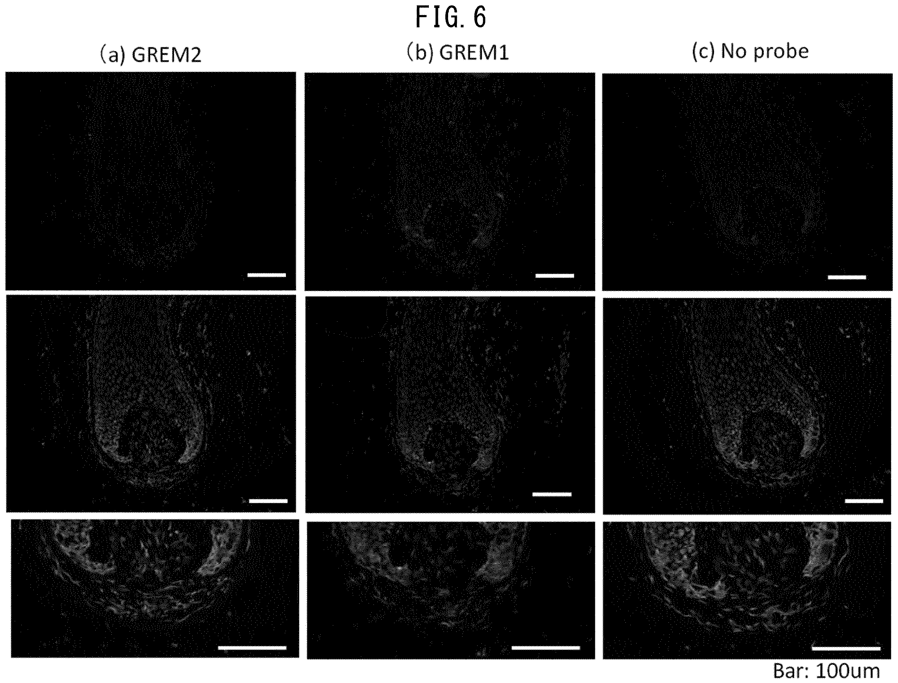

[FIG. 6] shows pictures illustrating the expression of *GREM2* and *GREM1* in human hair follicles and show the results of in *situ* hybridization. (a) *GREM2* probe, (b) *GREM1* probe, (c) no probe. Top row: *in situ* signal only; middle row: *in situ* signal and nucleus; bottom row: enlarged view of DSC portion. Red: *in situ* signal; Blue: nuclear staining with Hoechst 33342; Scale bar:100 µm.

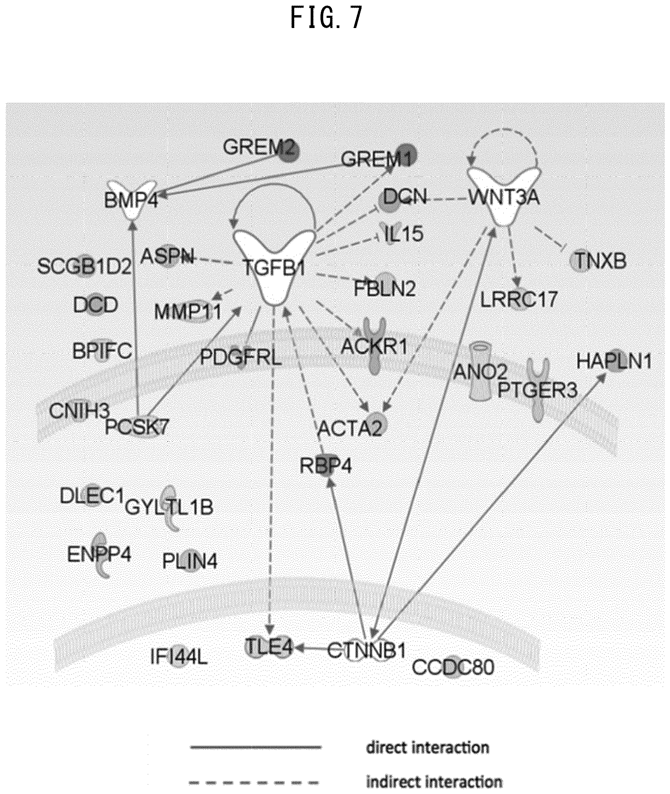

[FIG. 7] is a putative diagram illustrating the regulation of DSC marker genes and intracellular localization thereof.

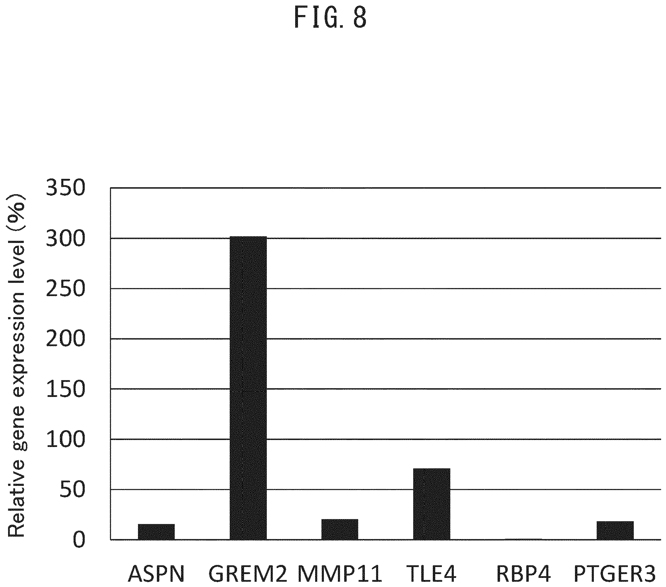

[FIG. 8] is a graph illustrating the change in expression levels of DSC marker genes before and after the dermal sheath cup (DSC) is cultured. Total RNA was recovered from intact DSCs and DSCs cultured *in vitro* for about 30 days and analyzed by quantitative PCR. Normalization was performed with GAPDH expression levels and the gene expression levels of DSC after culturing relative to when the intact DSC gene expression level is 100% is shown.

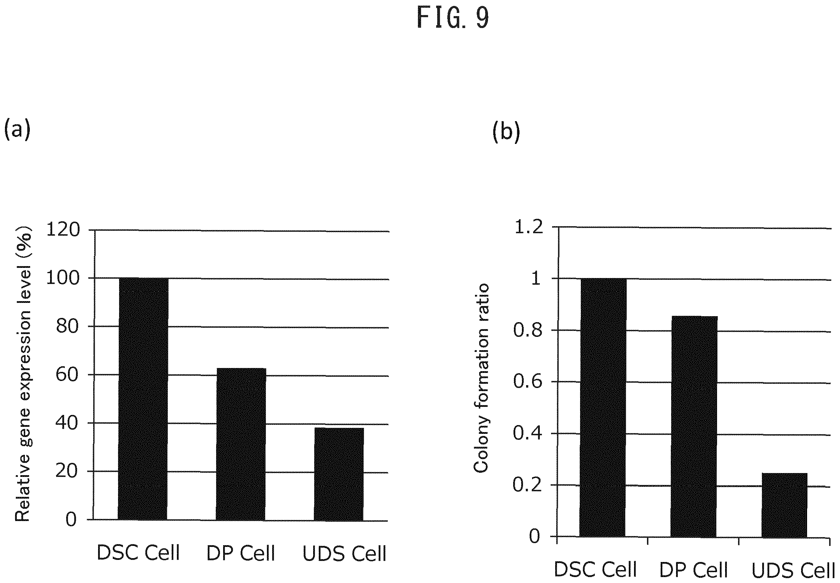

[FIG. 9] (a) is a graph illustrating *GREM2* expression levels in dermal sheath cup (DSC) cells, dermal papilla (DP) cells, and upper dermal sheath (UDS) cells for each cell after one passage. The graph illustrates the average value over two experiments of the GAPDH expression level in DSC cells relative to when the *GREM2* expression level is 100%. (b) is a graph comparing the colony forming capacity of dermal sheath cup (DSC), dermal papilla (DP), and upper dermal sheath (UDS) for each cell after culturing and one passage when the value of the DSC cell is one.

### DESCRIPTION OF EMBODIMENTS

Embodiments for carrying out the present invention are described below. However, the technical scope of the present invention is not limited to only the following embodiments.

The bulging portion at the deepest part of the hair within the skin is called a hair bulb and the part in the center of the hair bulb that is composed of mesenchymal cells is called a dermal papilla. The dermal papilla has nerves and capillaries therein, which supplies oxygen and nutrition from food and controls the development and growth of hair. There are hair matrix cells in places in contact with the dermal papilla and hair is produced at these sites. Namely, hair matrix cells take up nutrients and oxygen from capillaries within the dermal papilla and form hair by repeated cell division.

Herein, a "hair follicle (HF)" is defined as the tissue layer surrounding hair that contains epithelial cells such as hair matrix (hair matrix cells), inner root sheath, outer root sheath, dermal sheath, dermal papilla, and melanocytes, etc. Tissue or cells used herein may be derived from any animal but are preferably derived from vertebrates, more preferably derived from mammals, and most preferably derived from humans.

Herein, "dermal sheath (DS)" is the tissue comprising one or more dermal cell layers (vimentin-positive) surrounding the outermost layer of the hair follicle and includes α-smooth muscle actin- (α-SMA) positive cells. Dermal cells are continuous with the dermal papilla at the bottom most tip of the hair bulb. As will be described later, dermal sheath herein, includes the dermal sheath cup and the upper dermal hair root layer.

Herein, "dermal sheath cup (DSC)" refers to tissue located at the bottom of the hair bulb and is part of the dermal sheath (see Fig.1), and "dermal sheath cup (DSC) cells" are the cells that constitute the dermal sheath cup. It is known that dermal sheath cup cells are progenitor cells of dermal papilla cells and it is known that by transplanting dermal sheath cup cells into the skin, hair follicles are induced at the transplanted site. Namely, in a hair follicle-derived cell group or a composition for regenerating hair follicles containing a hair follicle-derived cell group, if it is possible to select a hair follicle-derived cell group or a composition which has a high proportion of dermal sheath cup cells and/or in which the activity thereof is high, then it would be possible to provide a hair follicle-derived cell group or a composition for regenerating hair follicles containing a hair follicle-derived cell group with a higher hair follicle inductive capability.

Herein, "upper dermal sheath (UDS)" refers to dermal sheath tissue from which the dermal sheath cup has been removed.

Herein, "hair follicle-derived cell group" refers to a cell group that contains cells that constitute the hair follicle. Furthermore, herein, the "composition for regenerating hair follicles containing a hair follicle-derived cell group" contains, other than the hair follicle-derived cell group, for example biocompatible substances. The biocompatible substances may include water, physiological saline, phosphate buffer, cell culture medium, biocompatible hydrogels (chitosan, collagen gel, gelatin, peptide gel, laminin gel, fibrin gel, etc.) but are not limited thereto.

As a result of extensive research carried out by the present inventors, genes specifically expressed in dermal sheath cup cells were discovered. Specifically, the gene or identifying marker used to identify dermal sheath cup cells in the present invention includes those listed below in Table 1.

**[Table 1]**

| Gene name | Gene Abbreviation | Accession number |
|---|---|---|
| aspor in | ASPN | NM_017680 |
| greml in 2, DAN family BMP antagonist | GREM2 | NM_022469 |
| matrix metallopeptidase 11 (stromelysin 3) | MMP11 | NM_005940 |
| prostaglandin E receptor 3 (subtype EP3) | PTGER3 | NM_198714, NM_198717 |
| retinol binding protein 4, plasma | RBP4 | NM_006744 |
| transducin-like enhancer of split 4 | TLE4 | NM_007005 |

Note that the specific sequences of the genes listed in Table 1 and Table 2 (see below) can be obtained from GeneBank (http://www.ncbi.nlm.nih.gov/). The specific gene sequences used in the present invention are not limited to sequences acquired using the accession numbers listed in Table 1 and Table 2. For example, a sequence identity of 70% or more, 75% or more, 80% or more, 85% or more, 90% or more, 91% or more, 92% or more, 93% or more, 94% or more, 95% or more, 96% or more, 97% or more, 98% or more, or 99% or more compared to the nucleotide sequences specified in Table 1 and Table 2 and splicing variants thereof are included. Furthermore, the genes may be derived from any animal but are preferably derived from vertebrates, more preferably derived from mammals, and most preferably derived from humans.

In one embodiment, the present invention includes the following aspect.

A method for identifying dermal sheath cup (DSC) cells in a hair follicle-derived cell group, characterized by using the expression level of the *GREM2* gene as an indicator.

In another embodiment, the present invention includes the following aspect.

A method for identifying dermal sheath cup (DSC) cells in a hair follicle-derived cell group, characterized by using the expression level of the ASPN gene as an indicator.

In another embodiment, the present invention includes the following aspect.

A method for identifying dermal sheath cup (DSC) cells in a hair follicle-derived cell group, characterized by using the expression level of the MMP11 gene as an indicator.

In another embodiment, the present invention includes the following aspect.

A method for identifying dermal sheath cup (DSC) cells in a hair follicle-derived cell group, characterized by using the expression level of the PTGER3 gene as an indicator.

In another embodiment, the present invention includes the following aspect.

A method for identifying dermal sheath cup (DSC) cells in a hair follicle-derived cell group, characterized by using the expression level of the RBP4 gene as an indicator.

In another embodiment, the present invention includes the following aspect.

A method for identifying dermal sheath cup (DSC) cells in a hair follicle-derived cell group, characterized by using the expression level of the TLE4 gene as an indicator.

In the hair follicle-derived cell group, by measuring the expression level of the aforementioned genes, the dermal sheath cup cells contained in the hair follicle-derived cell group can be identified. Furthermore, in the hair follicle-derived cell group, by measuring the expression level of the aforementioned genes, the hair follicle inductive effect of the hair follicle-derived cell group can be predicted.

In one embodiment, the present invention includes the following aspect.

A method for evaluating a composition for regenerating hair follicles containing a hair follicle-derived cell group, characterized by determining the proportion of dermal sheath cup (DSC) cells and/or the activity thereof in the hair follicle-derived cell group using the GREM1 gene expression level as an indicator.

In another embodiment, the present invention includes the following aspect.

A method for evaluating a composition for regenerating hair follicles containing a hair follicle-derived cell group, characterized by determining the proportion of dermal sheath cup (DSC) cells and/or the activity thereof in the hair follicle-derived cell group using the ASPN gene expression level as an indicator.

In another embodiment, the present invention includes the following aspect.

A method for evaluating a composition for regenerating hair follicles containing a hair follicle-derived cell group, characterized by determining the proportion of dermal sheath cup (DSC) cells and/or the activity thereof in the hair follicle-derived cell group using the MMP11 gene expression level as an indicator.

In another embodiment, the present invention includes the following aspect.

A method for evaluating a composition for regenerating hair follicles containing a hair follicle-derived cell group, characterized by determining the proportion of dermal sheath cup (DSC) cells and/or the activity thereof in the hair follicle-derived cell group using the PTGER3 gene expression level as an indicator.

In another embodiment, the present invention includes the following aspect.

A method for evaluating a composition for regenerating hair follicles containing a hair follicle-derived cell group, characterized by determining the proportion of dermal sheath cup (DSC) cells and/or the activity thereof in the hair follicle-derived cell group using the RBP4 gene expression level as an indicator.

In another embodiment, the present invention includes the following aspect.

A method for evaluating a composition for regenerating hair follicles containing a hair follicle-derived cell group, characterized by determining the proportion of dermal sheath cup (DSC) cells and/or the activity thereof in the hair follicle-derived cell group using the TLE4 gene expression level as an indicator.

By determining the proportion of dermal sheath cup (DSC) cells and/or the activity thereof in a hair follicle-derived cell group using the expression level of the aforementioned genes in a composition for regenerating hair follicles containing a hair follicle-derived cell group, the relative amount or activity of the dermal sheath cup cells contained in a hair follicle-derived cell group can be evaluated and as a result the hair follicle inductive effect of the dermal sheath cup cells can be predicted.

In another embodiment, the present invention comprises any of the aforementioned methods wherein the expression level of one or more genes selected from the group consisting of the *GREM2, ASPN, MMP11, PTGER3, RBP4,* and *TLE4* may further be used as an indicator. By combining these dermal sheath cup cell-specific genes, the more accurately the dermal sheath cup cells contained in the hair follicle-derived cell group or composition for regenerating hair follicles containing a hair follicle-derived cell group can be evaluated.

Furthermore, in another embodiment, the present invention in addition to the aforementioned step, further uses as an indicator, the expression level of one or more genes selected from the group consisting of the genes listed in Table 2 below *(GREM1 DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7).*

**[Table 2]**

| Gene name | Gene Abbreviation | Accession number |
|---|---|---|
| gremlin 1, DAN family BMP antagonist | GREM1 | NM_00372, NM_001191323 |
| decorin | DCN | NM_001920, NM_133507 |
| platelet-derived growth factor recep tor-like | PDGFRL | NM_006207 |
| atypical chemokine receptor 1 (Duffy blood group) | ACKR1 | NM_002036 |
| hyaluronan and proteoglycan link pro tein 1 | HAPLN1 | NM_001884 |
| dermcidin | DCD | NM_053283 |
| secretoglobin, family 1D, member 2 | SCGB1D2 | NM_006551 |
| actin, alpha 2, smooth muscle, aorta | ACTA2 | NM_001613 |
| perilipin 4 | PLIN4 | NM_001080400 |
| docking protein 6 | D0K6 | NM_152721 |
| ectonucleotide pyrophosphatase / pho sphodiesterase 4 (putative) | ENPP4 | NM_014936 |
| interleukin 15 | IL15 | NM_172175 |
| coiled-coil domain containing 80 | CCDC80 | NM_199511 |
| tenascin XB | TNXB | NM_019105 |
| fibulin 2 | FBLN2 | NM_001004019 |
| deleted in lung and esophageal cance r 1 | DLEC1 | NM_007335 |
| leucine rich repeat containing | LRRC17 | NM_001031692 NM_005824, MN_001031692 |
| zinc finger protein 791 | ZNF791 | NM_153358 |
| cornichon family AMPA receptor auxil iary protein 3 | CNIH3 | NM_152495 |
| proline rich 15-like | PRR15L | NM_024320 |
| DDB1 and CUL4 associated factor 4 | DCAF4 | NM_181340, NM_015604 |
| glycosyltransferase-like 1B | GYLTL1B | NM_152312 |
| uncharacterized protein BC008131 | L0C142937 | BC008131 |
| BPI fold containing family C | BPIFC | NM_174932 |
| anoctamin 2, calcium activated chlor ide channel | AN02 | NM_001278597 |
| interferon-induced protein 44-like | IFI44L | NM_006820 |
| proprotein convertase subtilisin / k exin type 7 | PCSK7 | NM_004716 |

Herein, "gene expression level" refers to what can be determined by detecting and measuring transcription products transcribed from any gene or translation products translated from transcription products used as templates. A transcription product is, for example, an RNA chain that has been transcribed from a gene DNA template, namely, RNA chains synthesized by RNA polymerase and RNA chains that have undergone intracellular post-transcriptional modification. An RNA chain included as transcription products is, for example, messenger RNA (mRNA). These RNA chains include those that have undergone intracellular post-transcriptional processing. Translation products are, for example, polypeptide chains that have been translated from transcription product templates that have been transcribed from genes, namely polypeptide chains synthesized by ribosomes and proteins formed through folding of these polypeptide chains. Translated products also include polypeptide chains and protein fragments.

The gene expression level can be measured using publicly known methods. For example, when transcription products of genes are measured to measure the gene expression level, suitable probes are designed in view of the target gene sequence, and by using these probes in methods such as quantitative PCR (qPCR), *in situ* hybridization, northern blot, and DNA microarrays, the gene expression level can be measured.

Further, when translation products of genes are measured to measure the gene expression level, by using antibodies that detect protein translated from target genes in methods such as western blot, flow cytometry (FACS), and ELISA, the gene expression level can be measured.

In one embodiment, the gene expression level can be compared by normalization (standardization) to the expression level of a suitable housekeeping gene. Housekeeping genes that can be used for comparison include, for example, GAPDH (glyceraldehyde-3-phosphate dehydrogenase), β-actin, β2-mycoglobulin, HPRT 1(hypoxanthine phosphoribosyltransferase 1), etc. The aforementioned genes used herein can be compared between different samples by this method by measuring the gene expression levels of a housekeeping gene and normalizing (standardizing) to the expression level of the housekeeping gene.

In one embodiment, "using gene expression levels as an indicator" may involve detecting the proportion of cells expressing genes at a predetermined level or more (e.g., at or above a signal from a negative control sample) in a hair follicle-derived cell group. Thereby, it is possible to evaluate the proportion of dermal sheath cup cells contained in the hair follicle-derived cell group.

In one embodiment, "using gene expression levels as an indicator" may involve detecting the expression level of a suitable gene from among the entire hair follicle-derived cell group and comparing the expression level of the same gene in a different sample. Thereby, it is possible to evaluate whether, for the entire hair follicle-derived cell group, there is high or low expression of the gene(s) expressed in dermal sheath cup cells.

In one embodiment, "the activity of dermal sheath cup cell (DSCC)" is the expression level of the aforementioned genes expressed in dermal sheath cup cells. By comparing the expression level of the aforementioned genes between different samples, the relative activity of the dermal sheath cup cells contained in hair follicle-derived cell groups can be compared.

In one embodiment of the present invention, a hair follicle-derived cell group cultured *in vitro* may be used. Thereby, it is possible to evaluate whether there have been any changes in the proportion or properties of dermal sheath cup cells contained in a hair follicle-derived cell group before and after culturing. Further, in another embodiment of the present invention, a hair follicle-derived cell group that has been subcultured at least once may be used. The hair follicle-derived cell group is cultured *in vitro* and subculturing may be carried out using a publicly known method and is not limited.

### EXAMPLES

The present invention will be described in more detail based on the following examples but is in no way limited thereby.

### <EXAMPLE 1>

### 1. Method

### 1-1. Preparation of hair follicle subunits

Strips of skin containing hair follicles (HF) were obtained from three females aged from 30 to 70, two of whom had undergone nevus-cell nevus excision and one a lipoma excision. Samples were collected from the margins of the excised skin so as to avoid the tumor nest. HF mesenchymal subunits from isolated intact HF were microdissected under a stereomicroscope (FIG. 1) with reference to McElwee K. J. et al. (Reference document 1) and Niiyama S. et al. (Reference document 2). In short, the HF was held with forceps at a place adjacent to the hair bulb region and incised by transection with a needle. The dermal sheath cup (DSC) of the hair bulb was inverted using forceps and a needle, the remaining epithelium-derived tissue was removed, and the dermal papilla (DP) was exposed. The DP was transected and separated from the DSC. The upper dermal sheath (UDS) was peeled off from the outer root sheath and separated with forceps while another pair of forceps was used to hold the HF at the proximal end.

### 1-2. Preparation of total RNA

Each of the subunits was pooled and dissolved in 1 ml of ISOGEN (Nippon Gene Co., LTD) and RNA extraction was performed according to the manufacturer's instructions. After precipitation, the quality of the RNA was evaluated from the ribosomal RNA ratio (28S/18S) using Agilent Bioanalyzer 2100 (Agilent Technologies, Palo Alto, CA USA). Samples having an RNA integrity number (RIN) of 6.5 to 8.3 were used for microarray analysis.

### 1-3. Microarray assay

Microarray analysis was carried out according to the manufacturer's instructions (Agilent technologies). 10 ng of total RNA was converted to fluorescently labelled cRNA by undergoing *in vivo* reverse transcription followed by transcription in the presence of oligo (dT) primer, Affinity Script reverse transcriptase, T7 RNA polymerase and CTP labelled with a Cy3 dye using low input quick amp labeling kit (Agilent Technologies). The resulting 600 ng of labelled cRNA was fragmented, hybridized with Agilent SurePrint G3 Human GE 8 × 60K microarray (G4851A, Agilent Technologies) and scanned with an Agilent dual laser microarray scanner (G2565AA). Signal intensities were quantified using Feature Extraction Software version 11.0.1.1 (Agilent Technologies). The data was processed for statistical analysis using Genespring GX 13.0 software (Agilent Technologies). The microarray data was recorded according to the Minimum Information About a Microarray Experiment (MIAME) guidelines and deposited in the Gene Expression Omnibus (GEO) database (accession number GSE95219; htttp://www.ncbi.nlm.nih.gov/geo).

### 1-4. In situ hybridization

In order to visualize the localization of *GREM2* and *GREM1* within cells, *in situ* hybridization was performed using paraffin embedded tissue section, *GREM2* and *GREM1* specific type 6 probe were used based on highly specific branched DNA signal amplification technology according to the manufacturer's manual for View RNA™ ISH Tissue 1-Plex Assay kit (Thermo Fisher Scientific Inc., Waltham, MA, USA). Fast Red substrate was used to detect signals and then fluorescence was observed. Nuclear staining was carried out using Hoechst 33342 (Thermo Fischer Scientific Inc.)

### 1-5. Quantitative PCR (qPCR)

Total RNA was extracted from each sample using NucleoSpin RNA XS (Takara Bio Inc., Shiga, Japan) according to the attached instruction manual. This was followed by cDNA preparation using CellAmp Whole Transciptome Amplification Kit (Real Time) Ver. 2 (Takara Bio Inc., Shiga, Japan).

qPCR was performed using LightCycler™ FastStart DNA MasterPLUS SYBR Green I and LightCycler (Roche Molecular Systems, Inc, Pleasanton) according to the attached instruction manual.

### 2. Results

### 2-1. Each HF subunit exhibits a different genetic profile.

The gene expression profiles of isolated intact HF subunits (DSC, DP, and UDS) were analyzed (FIG. 1). Microarray analyses of total RNA from microdissected HF subunits of three individual donors were performed to identify HF subunit-specific and distinguishable genes. Microarray probes for which the data was tagged unreliable for at least two individuals or equivalent to background, were excluded. As a result, 25,342 probes were used for analysis. In order to identify intact HF subunit-specific and distinguishable genes, 18,905 probes with a signal intensity of at least 50 with respect to at least one HF component were further selected for analysis.

First, principal component analysis (PCA) was performed and relationships between total microarray data sets were analyzed. The positions of the three HF subunits derived from the same individual were closely related along the PC1 dimension. However, it was clear that the positions of the same subunits derived from different individuals had a weak correlation along the PC3 dimension (FIG. 2). Considering these inter-individual differences, inter-individual comparisons were made for DSC versus DP, DSC versus UDS, and UDS versus DP and the cut off value was set to 1.5-fold. Although there were substantial inter-individual differences, it was clear from PCA that among the three individuals, not only were there similarity for DSC but also for DP and UDS profiles.

### 2-2. Extraction of DSC and DP marker genes

The number of genes which were commonly up-regulated or down-regulated in all three individuals is shown in Table 3.

**[Table 3]**

| | | Up-regulated | | |
|---|---|---|---|---|
| | | DSC | DP | UDS |
| Down-regulated | DSC | | 127 | 490 |
| | DP | 63 | | 659 |
| | UDS | 376 | 605 | |

The results for DSC versus DP identified 63 genes that had been up-regulated. Of these, genes that were also up-regulated in UDS and 5 probes which had not been characterized were eliminated, and 32 genes were obtained as DSC marker genes (Table 4).

**[Table 4]**

| Gene name | Gene Abbreviation | DSC/DP ratio |
|---|---|---|
| gremlin 2, DAN family BMP antagonist | GREM2 | 7 |
| gremlin 1, DAN family BMP antagonist | GREM1 | 6.6 |
| retinol binding protein 4, plasma | RBP4 | 4.9 |
| decor in | DCN | 3.7 |
| platelet-derived growth factor receptor-like | PDGFRL | 3.6 |
| atypical chemokine receptor 1 (Duffy blood group) | ACKR1 | 3.5 |
| hyaluronan and proteoglycan link protein 1 | HAPLN1 | 3.4 |
| dermcidin | DCD | 3.3 |
| asporin | ASPN | 2.9 |
| secretoglobin, family 1D, member 2 | SCGB1D2 | 2. 7 |
| act in, alpha 2, smooth muscle, aorta | ACTA2 | 2.6 |
| parilipin 4 | PLIN4 | 2.6 |
| docking protein 6 | D0K6 | 2.6 |
| ectonucleotide pyrophosphatase/phosphodiesterase 4 (putative) | ENPP4 | 2.6 |
| interleukin 15 | IL15 | 2.5 |
| coiled-coil domain containing 80 | CCDC80 | 2.3 |
| prostaglandin E receptor 3 (subtype EP3) | PTGER3 | 2.3 |
| tenascin XB | TNXB | 2.2 |
| fibulin 2 | FBLN2 | 2.1 |
| deleted in lung and esophageal cancer 1 | DLEC1 | 2.1 |
| matrix metal lopeptidase 11 (stromelysin 3) | MMP11 | 2 |
| leucine rich repeat containing 17 | LRRC17 | 2 |
| zinc finger protein 791 | ZNF791 | 1.9 |
| transducin-like enhancer of split 4 | TLE4 | 1.9 |
| cornichon family AMPA receptor auxiliary protein 3 | CNIH3 | 1.8 |
| proline rich 15-like | PRR15L | 1.7 |
| DDB1 and CUL4 associated factor 4 | DCAF4 | 1.7 |
| glycosyltransferase-like 1B | GYLTL1B | 1.7 |
| uncharacterized protein BC008131 | L0C142937 | 1.7 |
| BPI fold containing family C | BPIFC | 1.7 |
| anoctamin 2, calcium activated chloride channel | ANO2 | 1.7 |
| interferon-induced protein 44-like | IFI44L | 1.7 |
| proprotein convertase subtilisin/kexin type 7 | PCSK7 | 1.6 |

### 2-3 Characterization of DSC marker genes

The DSC marker gene list includes actin, alpha 2, smooth muscle (alpha-smooth muscle actin), prostaglandin E receptor 3 and matrix metallopeptidase 11, which have previously been reported to be expressed in the DS (reference documents 3 to 5). Seven extracellular matrix components, decorin, fibulin 2, coiled-coil domain containing 80, tenascin XB, asporin and hyaluronan and proteoglycan link protein 1 and matrix metallopeptidase 11, are also included in the list, and this category was shown to be significantly over-represented in gene ontology analysis. Most of the DSC marker genes were highly expressed, not only in the DSC, but also in the UDS, compared with the DP (FIG. 3). Thereamong, 6 genes, asporin, DAN family BMP antagonist gremlin 2 (*GREM* 2), matrix metallopeptidase 11, prostaglandin E receptor 3, retinol binding protein 4 and transducin-like enhancer of split 4 (*TLE4*) are specifically expressed in the DSC at higher levels, showing at least 1.5-fold up-regulation compared to the UDS (Table 5).

**[Table 5]**

| Gene name | Gene Abbreviation | Probe ID | Fold change (DSC/DP) | | | | Fold change (DSC/UDS) | | | |
|---|---|---|---|---|---|---|---|---|---|---|
| | | | ID1 | ID2 | ID3 | Average | ID1 | ID2 | ID3 | Average |
| asporin | *ASPN* | A_23_P216429 | 1.6 | 2.2 | 5.0 | 2.9 | 7.7 | 3.2 | 3.2 | 4.7 |
| gremlin 2, DAN family BMP antagonist | *GREM2* | A_24_P40626 | 1.6 | 1.8 | 17.6 | 7.0 | 13.2 | 4.9 | 7.0 | 8.4 |
| matrix metallopeptidase 11 (stromelysin 3) | *MMP11* | A_23_P57417 | 1.6 | 1.9 | 2.6 | 2.0 | 2.0 | 2.2 | 3.0 | 2.4 |
| prostaglandin E receptor 3 (subtype EP3) | *PTGER3* | A_33_P3265749 | 1.5 | 1.5 | 4.0 | 2.3 | 2.7 | 2.0 | 3.1 | 2.6 |
| retinol binding protein 4, plasma | *RBP4* | A_23_P75283 | 1.7 | 2.6 | 10.5 | 4.9 | 12.8 | 3.0 | 7.1 | 7.7 |
| transducin-like enhancer of split 4 | *TLE4* | A_33_P3302676 | 2.0 | 1.5 | 2.0 | 1.9 | 3.1 | 2.0 | 1.8 | 2.3 |

### 2-4. GREM2 is an intact DSC marker gene

6 DSC specific genes showed substantial up-regulation in DSC compared to both DP and UDS. From among these genes, quantitative PCR was performed for *GREM2* with intact DP and DSC tissues. The results clearly showed that DSC-specific up-regulation was almost 10 times higher than in DP (FIG. 4). In order to further confirm tissue-specific gene expression of the *GREM2* gene, *in situ* hybridization was carried out. The results were able to confirm that *GREM2* was being expressed within the DSC area of the hair bulb of hair follicles, and it was shown that *GREM2* expression is DSC specific within intact hair follicles (FIG. 5). Moreover, when *in situ* hybridization was performed with *GREM1* which belongs to the same *GREM* family, expression within HF was confirmed (FIG. 6).

### 2-5. Identification of upstream regulators for DSC marker genes by in silico analysis

The results of analyzing upstream regulators to evaluate changes in expression were consistent with the reported literature, and *TGFB1* was identified as a putative regulator of 9 DSC marker genes (FIG. 7). 5 genes are associated with the *WNT* pathway and one of these genes, *TLE4,* is a member of the Groucho family which are well-known nuclear components of the WNT pathway. The expression of the two *BMP* signaling antagonists *GREM1* and *GREM2* were also up-regulated in DSC. FIG 7 shows the putative regulatory network and intracellular localization of DSC signature factors and suggests that *TGFB1* regulates characteristic extracellular matrix components of DSC.

### <EXAMPLE 2>

DSC, which was isolated by the same method as in Example 1, was cultured for about 30 days *in vitro* at 37 °C in 5% CO₂ using Amnio Max culture medium (Thermofisher). Total RNA was recovered from intact DSC before and after culturing and qPCR was performed in the same manner as for the above section 1-5. Normalization was carried out with GAPDH expression levels and when intact DSC gene expression levels were 100% the DSC gene expression level after culturing was as illustrated in FIG. 8.

### <EXAMPLE 3>

### GREM2 is a marker gene for cultured DSC cells and is related to colony formation activity of cells

DSC, DP, and UDS which were isolated by the same method as in Example 1, were cultured *in vitro* at 37 °C in 5% CO₂ using Amnio Max culture medium (Thermofisher). Total RNA was collected from each cell that had been passaged once and qPCR was performed in the same manner as for the above section 1-5.

FIG. 9(a) shows relative values when the *GREM2* expression level is set to 100% with respect to the *GAPDH* expression level in DSC cells. The results confirm up-regulation of *GREM2* expression in DSC cells.

Colony formation of cultured cells was evaluated by a method comprising seeding 1000 cells in a 10 cm petri, culturing them for 12 days in Amnio Max culture medium, then counting the number of colonies that had formed. The results confirm that DSC cells have high colony forming activity (FIG. 9 (b)).

### <Reference Documents>

1. McElwee KJ, Kissling S, Wenzel E, Huth A, Hoffmann R. Cultured peribulbar dermal sheath cells can induce hair follicle development and contribute to the dermal sheath and dermal papilla. J Invest Dermatol 2003; 121: 1267-1275.
2.Niiyama S, Happle R, Hoffmann R. The feasibility of quantitative analysis of androgen metabolism by use of single dermal papillae from human hair follicles. Exp Dermatol 2001; 10: 124-127.
3. Jahoda CA, Reynolds AJ, Chaponnier C, Forester JC, Gabbiani G. Smooth muscle alpha-actin is a marker for hair follicle dermis in vivo and in vitro. J Cell Sci 1991; 99: 627-636.
4. Ishimatsu-Tsuji Y, Moro O, Kishimoto J. Expression profiling and cellular localization of genes associated with the hair cycle induced by wax depilation. J Invest Dermatol 2005; 125: 410-420.
5. Colombe L, Michelet JF, Bernard BA. Prostanoid receptors in anagen human hair follicles. Exp Dermatol 2008; 17: 63-72.

## Claims

1. A method for identifying dermal sheath cup (DSC) cells in a hair follicle-derived cell group, **characterized by** using the expression level of the *GREM2* gene as an indicator.

2. The method according to claim 1, **characterized by** further using the expression level of one or more genes selected from the group consisting of the *ASPN, MMP11, PTGER3, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.

3. The method according to claim 1 or 2 wherein the hair follicle-derived cell group has been cultured *in vitro.*

4. The method according to claim 3 wherein the hair follicle-derived cell group has been subcultured at least once.

5. A method for evaluating a composition for regenerating hair follicles containing a hair follicle-derived cell group, **characterized by** determining the ratio and/or activity of dermal sheath cup (DSC) cells in the hair follicle-derived cell group using the expression level of the *GREM2* gene as an indicator.

6. The method according to claim 5, **characterized by** further using the expression level of one or more genes selected from the group consisting of the *ASPN, MMP11, PTGER3, RBP4, TLE4, GREM1, DCN, PDGFRL, ACKR1, HAPLN1, DCD, SCGB1D2, ACTA2, PLIN4, DOK6, ENPP4, IL15, CCDC80, TNXB, FBLN2, DLEC1, LRRC17, ZNF791, CNIH3, PRR15L, DCAF4, GYLTL1B, LOC142937, BPIFC, ANO2, IFI44L* and *PCSK7* as the indicator.

7. The method according to claim 5 or 6 wherein the hair follicle-derived cell group has been cultured *in vitro.*

8. The method according to claim 7 wherein the hair follicle-derived cell group has been subcultured at least once.
